# EUROPÄISCHE PATENTSCHRIFT

(11) **EP 4 161 416 B1**
(45) Veröffentlichungstag und Bekanntmachung des Hinweises auf die Patenterteilung: **11.02.2026**
(21) Anmeldenummer: 21730889.9
(22) Anmeldetag: 02.06.2021
(51) Int. Cl.: A61B 17/70, A61B 17/86

(54) **KNOCHENANKER FÜR DIE OPTIMIERTE ZEMENTAPPLIKATION**
BONE ANCHOR FOR OPTIMIZED CEMENT APPLICATION
ANCRAGE OSSEUX POUR UNE APPLICATION DE CIMENT OPTIMISÉE

(30) Priorität: 03.06.2020 DE 102020003320
(43) Veröffentlichungstag der Anmeldung: 12.04.2023
(73) Patentinhaber: Mimeo Medical GmbH, 70794 Filderstadt (DE)
(72) Erfinder: HEUER, Frank, 70794 Filderstadt (DE)
(74) Vertreter: Stolmár & Partner Patentanwälte PartG mbB
(86) Internationale Anmeldenummer: PCT/EP2021/064855
(87) Internationale Veröffentlichungsnummer: WO 2021/245169

(56) Entgegenhaltungen:
- EP-A1- 3 210 554
- WO-A1-2020/008309
- DE-A1- 102011 017 602

## Beschreibung

### Stand der Technik

Osteoporose ist durch die Abnahme der strukturellen Integrität des Knochens gekennzeichnet. Häufig resultieren daraus Kompressionsfrakturen, die chirurgisch behandelt werden müssen. Aufgrund der reduzierten Knochenqualität besteht die klinische Herausforderung in der unzureichenden Verankerungsstabilität bei der Versorgung mit Knochenankern.

Aus DE3508759A1 ist eine Knochenschraube für die Behandlung von Femurfrakturen bekannt. Die Patent-Anmeldung beschreibt eine Knochenschraube mit einer mittig angeordneten Kanülierungsöffnung und mehreren seitlich verlaufenden Öffnungen, die dazu dienen, dass Knochenzement durch die Knochenschraube in den Knochen injiziert werden kann. Nachdem Aushärten des Knochenzements wird eine deutlich höhere Festigkeit zwischen Knochen und Knochenschraube erreicht.

Die WO 2020/008309 A1 offenbart ein Knochenimplantat, das mindestens ein Mittel zum Bereitstellen mindestens einer dynamischen Interaktion der Knochenstimulation für mindestens einen Bereich einer Knochenimplantat-Grenzfläche umfasst, die gebildet wird, wenn das Implantat in den Knochen eingesetzt wird.

Die EP 3 210 554 A1 offenbart einen Knochenanker umfassend einen Schaft mit einem freien Ende, einen Kanal, der sich bis zu dem freien Ende durch den Schaft erstreckt und eine Längsachse und mindestens eine Öffnung in einer Wand des Schafts aufweist, die den Kanal mit der Außenseite verbindet, wobei ein Verengungselement vorgesehen ist in dem Kanal in einem Bereich an oder nahe dem freien Ende, der einen Querschnitt des Kanals verengt, ohne den Kanal derart zu verschließen, dass Knochenzement oder eine andere in den Kanal eingeführte Substanz am Austritt durch das freie Ende gehindert wird.

Damit Knochenanker minimal-invasiv angewendet werden können, muss die Kanülierungsöffnung den Knochenanker vollständig durchqueren, damit der Knochenanker über einen Führungsdraht geführt in den Knochen eingebracht werden können. Werden zementierbare Knochenanker mit einer Durchgangsbohrung verwendet, besteht allerdings Gefahr, dass bei einer bikortikalen Verschraubung sich Knochenzement außerhalb des Knochens verteilen kann, was schwere Komplikationen hervorrufen kann.

Aus EP2140824A1 sind Knochenanker bekannt, die für die Versorgung mit Knochenzement so optimiert sind, dass sie nach dem minimal-invasiven Einbringen in den Knochen distal mit einem Stopfen verschlossen werden können, damit ein Zementaustritt verhindert wird. Nachteilig ist allerdings, dass ein solcher Mini-Stopfen als Extra-Komponente vorgehalten werden muss und das Handling eine ausreichende Sicherheit gegenüber dem unbeabsichtigten Verlust des Mini-Stopfens bieten muss. Ein einteiliger Aufbau ohne bewegliche Teile wäre deshalb wünschenswert.

Des Weiteren ist es notwendig, dass der Anwender die Injektionskanüle solang mit dem Knochenanker in Verbindung halten muss, bis der Zement vollständig ausgehärtet ist. Wird die Injektionskanüle frühzeitig entfernt, kann es aufgrund des hohen Applikationsdruckes dazu kommen, dass der Knochenzement rückwärtig aus dem Knochenanker ausläuft. Dies kann für Komplikationen im weiteren OP-Verlauf sorgen. Kein derzeit bekannter Knochenanker verhindert das rückwärtige Auslaufen des Zements, wenn eine Applikationskanüle direkt nach der Injektion abgezogen wird. Eine entsprechende Lösung hätte den großen Vorteil, einer deutlich schnelleren Applikation und einer signifikanten Materialersparnis bzgl. notwendiger Injektionskanülen. Denn beim herkömmlichen Verfahren der Zementaugmentation ist für jeden Knochenanker eine Injektionskanüle notwendig.

### Darstellung der Erfindung

Die erfindungsgemäße Lösung liegt in den Merkmalen des unabhängigen Anspruchs. Vorteilhafte Weiterbildungen der Erfindung sind Gegenstand der abhängigen Ansprüche.

Dies wird durch den erfindungsgemäßen Knochenanker (10) gelöst, wobei Knochenanker (10) zu einem Verbund oder Osteosynthesekonstrukt (1) von zwei oder mehr Knochenankern miteinander aufgebaut werden. Hierzu ist einschlägiger Stand der Technik vorhanden, weshalb hier nicht näher darauf eingegangen wird.

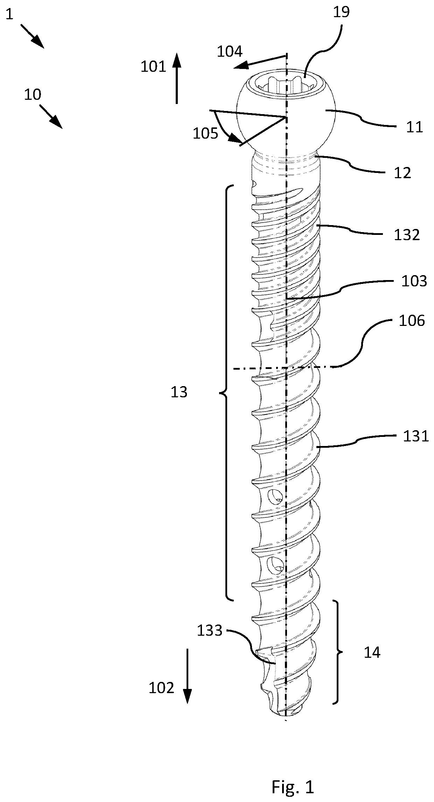

Für den erfindungsgemäßen Knochenanker (10), sind raumzuweisende Koordinatenreferenzen definiert, wie zum Beispiel die proximale Richtung (101), die distale Richtung (102), die sich entlang einer Zentralachse (103) ausdehnen. Von der Zentralachse (103) nach außen abgehend definiert sich die radiale Ausbreitung (104). Die umfängliche Ausbreitung (105) ist durch einen konstanten Radius und entlang eines variablen Umfangswinkels definiert (Fig. 1). Des Weiteren existiert eine Mittenebene (106), welche die Raumrichtungen distal (102) und proximal (10) gleichermaßen voneinander trennt.

In einer ersten Ausführungsform wird ein Knochenanker (10) für die Fixation von Knochenkomponenten und Knochenfragmenten beschrieben, welcher aus einem Schaft (13), einem Halsbereich (12) und einen in proximaler Richtung (10) befindlichen Kopf (11), sowie in distaler Richtung (102) befindlicher Spitze (14) besteht.

Ergänzend ist zu erwähnen, dass der Knochenanker (10) optimalerweise einstückig ausgebildet ist und mit Hilfe eines additiven Verfahrens hergestellt wird (das Herstellungsverfahren wird nicht beansprucht).

Kann der Knochenanker (10) nicht mit einem additiven Verfahren hergestellt werden, eignet sich ein mehrteiliger Aufbau des Knochenankers, welcher auf irgendeine dem Stand der Technik bekannten Methode zusammengefügt wird. Der Kopf (11) ist vorzugsweise als Linse, Schrägkopf oder als Kugelkopf ausgebildet. Es sind aber auch eine Zusammensetzung aus unterschiedlichen Rundungen und Fläche denkbar. Hauptmerkmal des Kopfes ist es, dass der Kopf (11) einen größeren Außendurchmesser als der Halsbereich (12) aufweist. Vorzugsweise besitzt der Knochenanker eine Werkzeugansatzstelle (19), die zur Einleitung eines Drehmoments geeignet ist. Zur minimal-invasiven Versorgung ist es vorteilhaft, wenn der Knochenanker eine vollständig durchquerende Kanülierungsöffnung (15, 16, 40, 20, 30) besitzt, durch die ein chirurgischer Führungsdraht geführt werden kann.

Als Knochenanker kommen vorzugsweise Knochenschrauben zum Einsatz, die mit einem Knochen verschraubbar sind. Es sind aber auch Haken, Klemmen, Nägel und andersartig gestaltete Knochenanker anwendbar. In dem hier aufgeführten Beispiel eines Knochenankers (10), wird eine Knochenschraube mit einem Schaft (13) und einen am Schaft befindlichen Knochengewinde (131) vorgestellt. Das Gewindegewinde (131) kann abschnittsweise eine feinere Verzahnung (132) aufweisen, welches für einen härteren Kortikal-Knochen besser geeignet ist. Vorteilhaft ist ein zulaufendes Gewinde (132) mit einer Schnittkante (133) an der Knochenankerspitze (14), damit sich der Knochenanker beim Einschrauben selbstschneidend in den Knochen ziehen kann.

Bei schwachem Knochen, wie beispielsweise bei einer Osteopenie oder Osteoporose, kann es notwendig sein, den Knochenanker zusätzlich zu augmentieren. Dies kann mit Knochenzement erfolgen. Knochenzement ist vorzugsweise ein Polymer, welches aus mindestens zwei Komponenten vermischt und im flüssigen bzw. pasten-ähnlichem Zustand injiziert wird. der Knochenzement härtet nach wenigen Minuten im Knochen zu einem Kunststoff aus und verbindet sich mit der schwamm-artigen Knochenstruktur. Angewandt wird meist ein Polymethylmethacrylat-Zement. Alternativ sind auch andere Medien für die Abgabe durch den Knochenanker denkbar. Es ist denkbar, dass alternative Medien, wie beispielsweise pharmazeutisch wirkende Medien, oder Medien mit Zellen, Nährstoffe, oder Medien die als Erbinformationsträger dienen, oder Impfstoffe durch den Knochenanker verabreicht werden. Deshalb wird hier in dieser Schrift fortwährend das injizierbare Medium einfach als Flüssigkeit (17) bezeichnet.

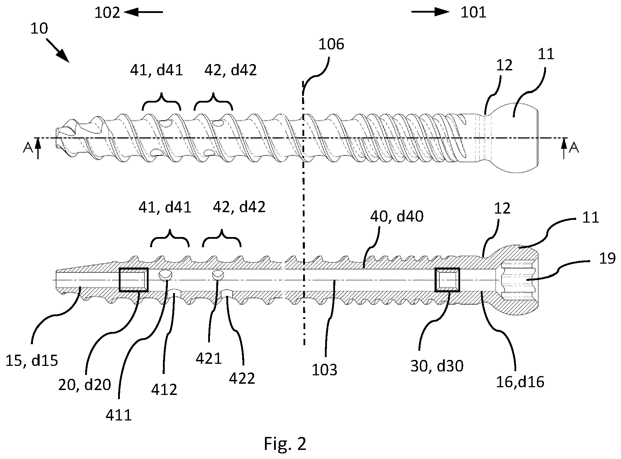

Im Inneren des Knochenankers (10) befindet sich eine hauptsächlich zylindrisch geformte Hohlkammer (40), die sich entlang der Zentralachse (103) erstreckt (Fig. 2), wobei die Hohlkammer (40) an eine proximal (101) von einer Mittenebene (106) aus gelegene Transitionszone (30) angrenzt. Diese Transitionszone (30) besitzt wenigstens abschnittsweise einen Innendurchmesser (d30) und die Hohlkammer (40) besitzt wenigstens abschnittsweise einen Öffnungsdurchmesser (d40). Dabei ist der Öffnungsdurchmesser der Hohlkammer (d40) größer ist als der Innendurchmesser (d30) der proximalen Transitionszone (30). Durch diesen Querschnittsunterschied (Vgl. d40 und d30) wird ein Zurückströmen der Flüssigkeit (17) erschwert, wenn sie sich bereits in der Hohlkammer (40) befindet.

Die Hohlkammer (40) ist dabei in distaler-proximaler Ausrichtung hauptsächlich zentral im Inneren des Knochenankers (10) vorgesehen. Wie eingangs beschrieben, ist es vorteilhaft, dass die Hohlkammer (40) so gestaltet ist, dass sie sich wenigstens abschnittsweise mit einem konstanten Öffnungs-Durchmesser (d40) entlang der Zentralachse (103) erstreckt und dass die Hohlkammer (40) an mindestens eine Transitionszone (20, 30) angrenzt. Die Hohlkammer (40) besitzt mindestens eine seitlich abgehende und mit der Hohlkammer kommunizierende Öffnung (411, 412, 421, 422). Vorzugsweise sind die Öffnungen in Umfangsrichtung in ring-artiger Formation (41 oder 42) angeordnet. Bei mehr als einer in Umfangsrichtung ring-artiger Öffnungs-Formation (41 und 42), haben die Öffnungen je Formation unterschiedliche Öffnungsdurchmesser. Bei in einen Knochen eingeschraubten Knochenanker (10), kommunizieren die seitlichen Öffnungen von der Hohlkammer (40) aus mit dem umgebenden Knochengewebe. Sie sind dazu geeignet, dass die Flüssigkeit (17), die in den Knochenanker (10) injiziert wird, durch die seitlichen Öffnungen in das umliegende Gewebe abgegeben wird. Ein unterschiedlicher Durchmesser der Öffnungs-Formationen (41, 42) hat den Vorteil, dass aufgrund des lokalen Druckunterschieds innerhalb der Flüssigkeit (17), durch alle Öffnungen (411, 412, 421, 422) ein ähnlicher Volumenstrom generiert wird. Dies wird dadurch erreicht, dass die Öffnungen (421, 422, 42), die näher zur proximalen Transitionszone (30) liegen, einen kleineren Durchmesser besitzen als die Öffnungen (411, 412) der Formation (41), die weiter distal liegen.

Im weiteren proximalen Verlauf (101) entlang der Zentralachse (103), d.h. nach der proximalen Transitionszone (30) befindet sich eine Öffnung (16), die dazu geeignet ist, eine Kanüle wenigstens abschnittsweise aufzunehmen (Fig. 2). Der Öffnungsdurchmesser (d16) der an die proximale Transitionszone (30) angrenzenden Öffnung (16) ist größer als wenigstens ein Abschnitt des Innendurchmessers (d30) der proximalen Transitionszone (30). Des Weiteren ist es vorteilhaft, wenn die die Öffnung (16) nach proximaler Richtung (101) in der Werkzeugansatzstelle (19) mündet.

Um das Risiko eines unbeabsichtigten Austretens der Flüssigkeit (17) nach distal (102) zu reduzieren, ist es von Vorteil, dass die Hohlkammer (40) ebenfalls an eine distal (102) von einer Mittenebene (106) aus gelegene Transitionszone (20) angrenzt, und die distale Transitionszone (20) wenigstens abschnittsweise einen Innendurchmesser (d20) besitzt und die Hohlkammer (40) wenigstens abschnittsweise einen Öffnungsdurchmesser besitzt (d40), und der Öffnungsdurchmesser der Hohlkammer (d40) größer ist als der Innendurchmesser (d20) der distalen Transitionszone (20). Dieser Durchmesserunterschied (d20, d40) hat zur Folge, dass die Flüssigkeit (17) bevorzugt durch die zuvor erwähnten seitlichen Öffnungen (411, 412, 421, 422) an das umliegende Gewebe abgeleitet wird, ohne dass sie nach distal (102) durch eine distale Öffnung (15) austreten kann. Vorzugsweise besitzt die distale Öffnung (15) einen kleineren Durchmesser (d15) als der Innendurchmesser der Hohlkammer (d40), wobei es vorteilhaft ist, dass der Durchmesser der distalen Öffnung (d15) in etwa gleichgroß dem wenigstens abschnittsweise vorhandenen Innendurchmessers der distalen Transitionszone (20) ist. Damit erfährt ein eingeführter Führungsdraht am distalen Eingang (15) keine weiteren Durchmesseränderungen.

Wie bereits erwähnt, grenzt die Hohlkammer (40) direkt an mindestens eine Transitionszone (20, 30). Die Transitionszone besitzt wenigstens abschnittsweise einen kleineren Öffnungsdurchmesser (d20, d30) als die Hohlkammer (d40). Durch den reduzierten Öffnungsdurchmesser (d20, d30) wird im Bereich der Transitionszone (20, 30) der Staudruck einer durchzwängten Flüssigkeit (17) erhöht. Dadurch entsteht für die Flüssigkeit (17) eine Überwindungsstelle was
einer teildurchlässigen Barriere gleichzusetzen wäre. Messbare wäre dies beispielsweise über den Strömungswiderstand. Der Strömungswiderstand ist beeinflussbar, durch einen Druckunterschied in der Flüssigkeit (17), der inneren Reibung, der Viskosität und der Volumenstrom der Flüssigkeit (17). Strukturell kann der Strömungswiderstand durch die Oberflächen-Reibung, einer OberflächenRauheit, dem Durchmesser und der Länge des zu überwindenden Streckabschnitts beeinflusst werden.

Aufgabe einer erfindungsgemäßen Transitionszone (20, 30) ist es, sobald sich die Flüssigkeit (17) in der Hohlkammer (40) befindet, dass die Flüssigkeit (17) bevorzugt durch die seitlichen Öffnungen (41, 42) abfließt und das Risiko des unbeabsichtigten Durchquerens der Flüssigkeit (17) durch die Transitionszonen (20, 30) minimiert ist. Deshalb ist es die Aufgabe der Transitionszone (20, 30) einen Streckenabschnitt bereitzustellen, welcher im Vergleich zur Hohlkammer (40) abschnittsweise einen hohen Strömungswiderstand innerhalb der Transitionszone (20, 30) erzeugt. Vorzugsweise soll der Strömungswiderstand von seiner Wirkung richtungsabhängig (21, 22) von der Flussrichtung der Flüssigkeit (17) sein.

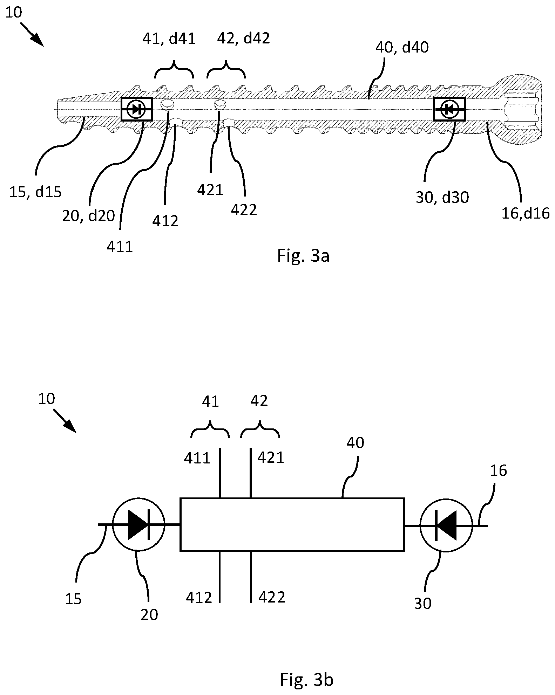

In einer bevorzugten Ausgestaltungsform übt wenigstens eine Transitionszone (20 und/oder 30) einen Strömungswiderstand aus, oder erzeugt einen Druckunterschied, oder beeinflusst die inneren Reibung der Flüssigkeit (17), oder erzeugt eine höhere Oberflächen-Reibung, oder beeinflusst den Volumenstrom; wobei der oder die Unterschiede in einer Sperr-Richtung (22) höher sind als in einer Durchlass-Richtung (21). Aus diesem Aufbau resultiert eine Art fluidische Diode, was mit einem Dioden-Symbol in den folgenden Figuren dargestellt ist (Fig. 3a). Es ist dadurch möglich zwischen einer Durchlass-Richtung (21) und einer Sperr-Richtung (22) zu unterscheiden. Wobei es wichtig ist zu erwähnen, dass in Sperr-Richtung (22) der Flüssigkeitsstrom nicht zu 100% unterbunden werden kann, da zentral in der Transitionszone (20, 30) immer eine Öffnung (d20, d30) vorhanden ist. Vielmehr kann mit einer Transitionszone das Risiko eines unbeabsichtigten Austretens reduziert oder nicht vollständig eliminiert werden.

In Fig. 3a ist zu erkennen, dass die Hohlkammer (40) von einer Mittenebene (106) aus betrachtet nach distal an eine Transitionszone (20) und proximal an eine Transitionszone (30) angrenzt und die distale Transitionszone (20) eine Durchlass-Richtung (21) in proximaler Richtung (101) und die proximale Transitionszone (30) eine Durchlass-Richtung (21) in distaler Richtung (102) aufweist. Ebenfalls zu erkennen ist auch, dass die Hohlkammer (40) von einer Mittenebene (106) aus betrachtet nach distal an eine Transitionszone (20) und proximal an eine Transitionszone (30) angrenzt und die distale Transitionszone (20) eine Sperr-Richtung (22) in distaler Richtung (101) und die proximale Transitionszone (30) eine Sperr-Richtung (22) in proxiamler Richtung (102) besitzt.

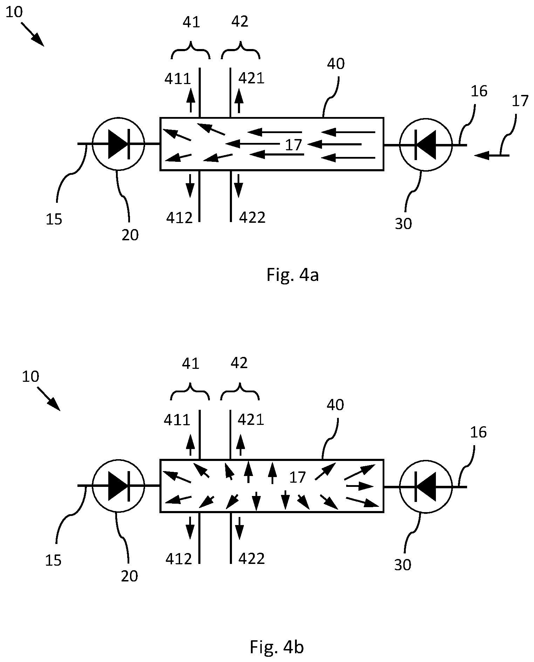

Das Zusammenspiel der beiden fluidischen Dioden (20, 30) ist in den Figuren 3b, 4a und 4b illustriert. Fig. 3b zeigt einen vereinfachten modellhaften Aufbau des Knochenankers. Die Hohlkammer (40) ist als Behälter dargestellt, welche an die beiden in ihrer Durchlass-Richtung gegenläufig angeordneten fluidischen Dioden (20 und 30) angrenzt. Es ist ein Zulauf vorgesehen (16), wodurch eine Kanüle eine Flüssigkeit (17) injiziert kann. Die Hohlkammer (40) besitzt mindestens einen Ablauf, was durch die seitlichen Öffnungen (41, 42, 411, 412, 421, 422) vereinfacht dargestellt ist. Zur Vollständigkeit ist die distale Öffnung (15) vereinfacht als Ableitung dargestellt. Dem Schaubild Fig. 4a kann entnommen werden, wie sich der Flüssigkeitsstrom gestaltet, sobald eine Flüssigkeit (17) durch die Zuleitung (16) injiziert wird. Die proximale fluidische Diode (30) ist in Durchlass-Richtung und lässt die Flüssigkeit (17) in die Hohlkammer (40) passieren. Die distale fluidische Diode (20) ist in Sperr-Richtung angeordnet und verhindert ein Auslaufen der Flüssigkeit nach distaler Richtung (102). Die Flüssigkeit kann durch die seitlichen Öffnungen (41, 42 etc.) entweichen.

Wird die Injektionskanüle abgezogen, stellt sich ein anderes Szenario dar (Fig. 4b). Der Eingangsstrom über die Öffnung (16) wird unterbrochen. Es verbleibt die Flüssigkeit einzig im der Hohlkammer (40). Das umliegende Knochengewebe sorgt dafür, dass ein hydrostatischer Druck in der Hohlkammer (40) aufrechterhalten bleibt. Beide fluidischen Dioden (20, 30) sind nun in Sperr-Richtung aktiv und reduzieren einen Flüssigkeitsaustritt entlang der Zentralachse (103). Die Flüssigkeit (17) kann nur durch die seitlichen Öffnungen (41, 42 etc.) entweichen.

Zusammengefasst heißt dies, die proximale Transitionszone (30) verhindert oder reduziert ein Zementaustreten nach proximal (101). Sobald der Anwender die Injektionskanüle entfernt, wird ein Druckabbau der Hohlkammer (40) über den proximalen Zugang (16) verhindert werden. Die distale Transitionszone (20) verhindert ein Zementaustreten nach distal (102), damit kein Knochenzement ins umliegende Gewebe austreten kann.

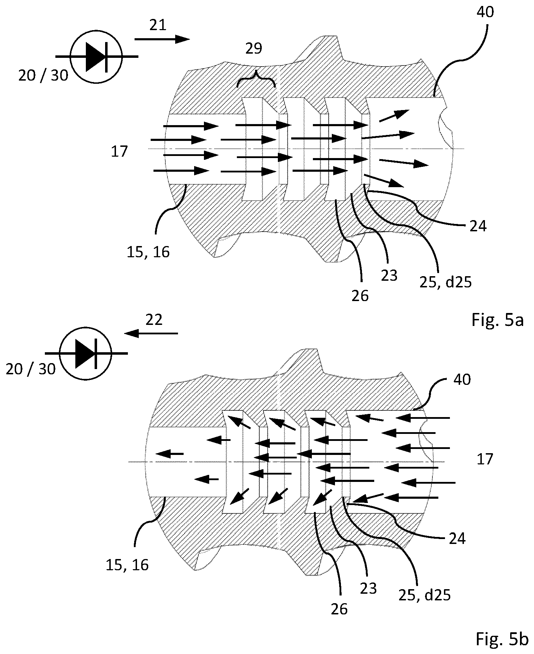

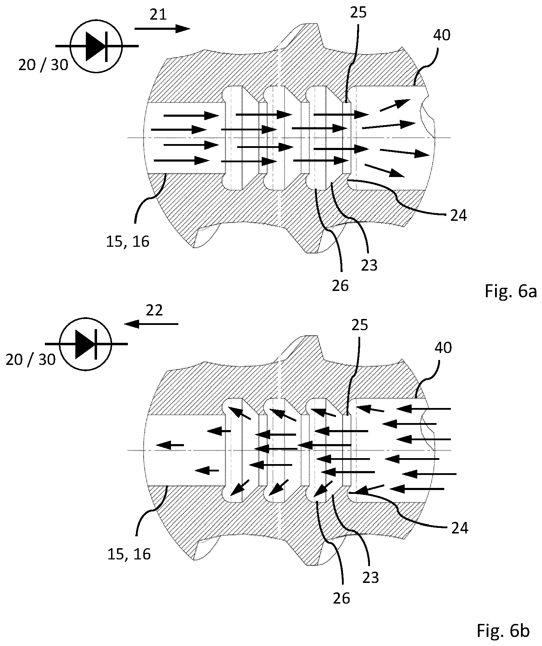

Der einfachste Aufbau einer fluidischen Diode kann mit Hilfe von nach radial innen-gerichteten Flächenelementen (24, 34) erzeugt werden. Die Transitionszone (20, 30) besteht aus wenigstens einem Segment (29), welches wenigstens ein Flächenelement (25, 35, 272, 273) besitzt, was den inneren Öffnungsdurchmesser (d20, d30) definiert, und wenigstens ein Flächenelement (26, 36), welches einen Stauraum definiert, und wenigstens ein Flächenelement (24, 34) besitzt, welches in Sperr-Richtung (22) einen Staudruck erzeugt, in dem das Flächenelement (24, 34) in einen Winkel zu einer Orthogonale der Zentralachse (103) zwischen -20° und 20° angeordnet ist. Die sperrenden Flächenelemente (24, 34) erzeugen einen Widerstand, der zur lokalen Erhöhung des Staudrucks führt und somit einen Rückstau in der Flüssigkeit (17) generiert und das Durchdringen der Flüssigkeit durch die Transitionszone (20, 30) erschwert wird. Die sperrenden Flächenelemente können dabei planar verlaufen (Fig. 5a, b), oder konkav (Fig. 6a, b), oder konvex gekrümmt sein, oder polygonale Elemente besitzen. Dabei ist es vorteilhaft, wenn sich ein solche Anordnung von Flächenelementen und deren Konfigurationen entlang der Zentralachse (103) segmentweise (29) wiederholen. Damit wird ein Verstärkungseffekt der Sperr-Richtung (22) erzeugt. Zur Vollständigkeit sind noch Flächenelemente (23, 33) zu erwähnen, die eine Durchlass-Richtung (21) einer Flüssigkeit (17) begünstigen. Sie sorgen nicht für einen erhöhten Staudruck im Vergleich zur Sperr-Richtung (22). Die Flächenelemente (z.B. 23, 24, 25, 26) sind dabei vorzugsweise als um die Zentralachse (103) in Umfangsrichtung (105) umlaufende rotatorische Austragungen vorgesehen. Alternativ können die Flächenelemente auch in einer Helix entlang der Zentralachse (103) ausgetragen werden. Damit können die Flächenelemente auch klassisch drehend oder fräsend hergestellt werden.

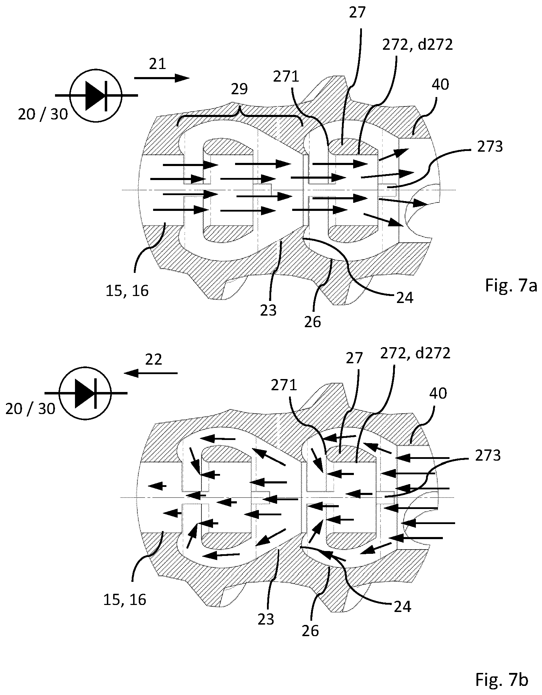

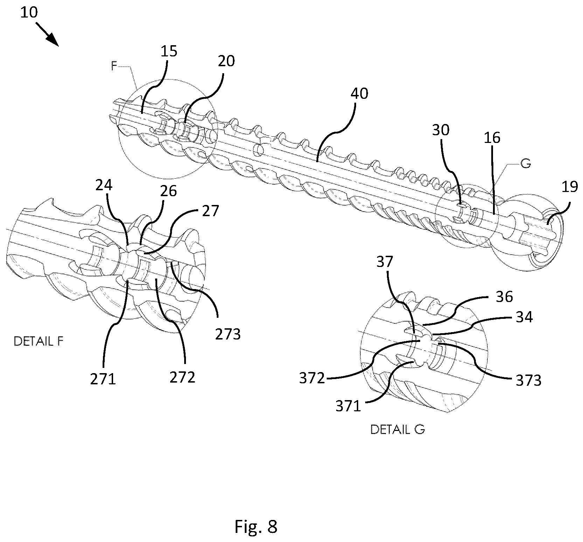

In einer alternativen Ausgestaltungsform sind fluidische Dioden (20, 30) vorgesehen, die einen dynamischen Einfluss auf den Strömungsfluss der Flüssigkeit (17) ausüben und somit wirkungsvoller sind. Optimalerweise besitzen die sperrenden Flächenelemente (24, 34) wenigstens eine konkave Krümmung (Fig. 7a, b und Fig. 8). Damit die Flüssigkeit sich nicht daran aufstaut und dort verharrt, ist es vorteilhaft, wenn ein kontinuierlicher Flüssigkeitsstrom generiert wird, welcher permanent den Staudruck erhöht und dem Hauptstrom der Flüssigkeit entgegenwirkt oder ändert. Dies kann dadurch erreicht werden, indem die Transitionszone (20, 30) wenigstens aus einem Segment (29) besteht, welches wenigstens ein Flächenelement (26, 36) besitzt, welches einen Stauraum definiert, und in diesem Stauraum ein Ring (27) darin angeordnet ist. Der Ring (27) befindet sich innerhalb einer konzentrischen Austragung (26, 36) und ist von Trägerelementen (273, 373) gehalten, die in Verbindung zur Außenwandung (13) des Knochenankers (11) stehen. Zwischen den Trägerelementen, Außenwandung und Ring befinden sich Zwischenräume, die zur Umleitung des Flüssigkeitsstroms dienen.

Der Ring (27, 37) definiert dabei mit seiner Innenseite (272, 273) den Öffnungsdurchmesser der Transitionszone (d20, d30). Optimalerweise ist der Ring (27, 37) an seiner radialen Außenseite (271, 371) konvex gekrümmt ist. Die konvexe Krümmung sorgt für einen Coanda-Effekt in der Flüssigkeit, so dass die Flüssigkeit nicht gestaut, sondern in den Hauptstrom umgeleitet wird.

### Kurze Beschreibung der Zeichnungen zeigen

Fig. 1 eine Schrägansicht des erfindungsgemäßen Knochenankers,
Fig. 2 Seitenansicht und dazu gehöriger Schnittansicht durch den erfindungsgemäßen Knochenanker,
Fig. 3a weitere Schnittansicht, mit der vereinfachten Darstellung einer fluidischen Diode,
Fig. 3b Modell-hafte Darstellung der Fluid-Strom-relevanten Komponenten,
Fig. 4a in Anwendung bei Applikation einer Flüssigkeit durch eine Kanüle, und
Fig. 4b unmittelbar nachdem die Kanüle entfernt wurde.
Fig. 5a zeigt einen Aufbau einer einfachen Form einer fluidische Diode in Durchlass-Richtung, und Fig. 5b in Sperr-Richtung.
Fig. 6a stellt Aufbau einer alternativen Form einer fluidische Diode in Durchlass-Richtung, und Fig. 6b in Sperr-Richtung dar.
Fig. 7a illustriert eine alternative Ausgestaltungsform einer fluidischen Diode in Durchlass-Richtung, wobei Fig. 7b das GegenstromPrinzip darstellt.
Fig. 8 zeigt einen ¾ Schnitt in einer Schrägansicht aus Fig. 7a/b.

## Patentansprüche

1. Knochenanker (10) für die Fixation von Knochenkomponenten und Knochenfragmenten, bestehend aus einem Schaft (13), einem Halsbereich (12) und einen in proximaler Richtung (101) befindlichen Kopf (11), sowie in distaler Richtung (102) befindlicher Spitze (14), wobei der Knochenanker (10) eine hauptsächlich zylindrisch geformte Hohlkammer (40) mit einer seitlich abgehenden und mit der Hohlkammer kommunizierenden Öffnung (411, 412, 421, 422), wobei die Hohlkammer (40) sich entlang der Zentralachse (103) erstreckt, besitzt, wobei die Hohlkammer (40) an eine proximal (101) von einer Mittenebene (106) aus gelegene Transitionszone (30) angrenzt, **dadurch gekennzeichnet**, dassdie Hohlkammer (40) in distaler-proximaler Ausrichtung hauptsächlich zentral im Inneren des Knochennkers (10) vorgesehen ist und die Transitionszone (30) wenigstens abschnittsweise einen Innendurchmesser (d30) besitzt und die Hohlkammer (40) wenigstens abschnittsweise einen Öffnungsdurchmesser besitzt (d40), und der Öffnungsdurchmesser der Hohlkammer (d40) größer ist als der Innendurchmesser (d30) der proximalen Transitionszone (30).

2. Knochenanker nach Anspruch 1, **dadurch gekennzeichnet, dass** im weiteren proximalen Verlauf (101) entlang der Zentralachse (103) eine Öffnung (16) an die proximale Transitionszone (30) angrenzt, die dazu geeignet ist, eine Kanüle wenigstens abschnittsweise aufzunehmen.

3. Knochenanker (10) nach Anspruch 2, **dadurch gekennzeichnet, dass** der Öffnungsdurchmesser (d16) der an die proximalen Transitionszone (30) angrenzenden Öffnung (16) größer ist als wenigstens ein Abschnitt des Innendurchmessers (d30) der proximalen Transitionszone (30).

4. Knochenanker (10) nach einem der vorhergehenden Ansprüche, **dadurch gekennzeichnet, dass** die Öffnung (16) nach proximaler Richtung (101) in einer Werkzeugansatzstelle (19) mündet.

5. Knochenanker (10) nach einem der vorhergehenden Ansprüche, **dadurch gekennzeichnet, dass** die Hohlkammer (40) an eine distal von einer Mittenebene (106) aus gelegene Transitionszone (20) angrenzt, und die distale Transitionszone (20) wenigstens abschnittsweise einen Innendurchmesser (d20) besitzt und die Hohlkammer (40) wenigstens abschnittsweise einen Öffnungsdurchmesser besitzt (d40), und der Öffnungsdurchmesser der Hohlkammer (d40) größer ist als der Innendurchmesser (d20) der distalen Transitionszone (20).

6. Knochenanker (10) nach einem der vorhergehenden Ansprüche, **dadurch gekennzeichnet, dass** die Transitionszone (20, 30) einen Streckenabschnitt definiert, der im Vergleich zu einem gleichlangen Streckenabschnitt der Hohlkammer (40), einen höheren Strömungswiderstand hervorruft.

7. Knochenanker (10) nach einem der vorhergehenden Ansprüche, **dadurch gekennzeichnet, dass** die Transitionszone (20, 30) einen Streckenabschnitt definiert, der im Vergleich zu einem gleichlangen Streckenabschnitt der Hohlkammer (40), einen höheren Druckunterschied in der Flüssigkeit (17) hervorruft.

8. Knochenanker (10) nach einem der vorhergehenden Ansprüche, **dadurch gekennzeichnet, dass** die Transitionszone (20, 30) einen Streckenabschnitt definiert, der im Vergleich zu einem gleichlangen Streckenabschnitt der Hohlkammer (40), zu einer höheren inneren Reibung in der Flüssigkeit (17) führt.

9. Knochenanker (10) nach einem der vorhergehenden Ansprüche, **dadurch gekennzeichnet**, die Transitionszone (20, 30) einen höheren Oberflächen-Reib-Koeffizienten oder eine höhere OberflächenRauheit besitzt als die Hohlkammer (40).

10. Knochenanker (10) nach einem der vorhergehenden Ansprüche, **dadurch gekennzeichnet, dass** die Transitionszone (20, 30) einen kleineren Volumenstrom der Flüssigkeit (17) erlaubt als die Hohlkammer (40).

11. Knochenanker (10) nach einem der vorhergehenden Ansprüche, **dadurch gekennzeichnet, dass** die Transitionszone (20, 30) wenigstens aus einem Segment (29) besteht, welches wenigstens ein Flächenelement (25, 35, 272, 273) besitzt, was den inneren Öffnungsdurchmesser (d20, d30) definiert, und wenigstens ein Flächenelement (26, 36), welches einen Stauraum definiert, und wenigstens ein Flächenelement (24, 34) besitzt, welches in Sperr-Richtung (22) einen Staudruck erzeugt, in dem das Flächenelement (24, 34) in einen Winkel zu einer Orthogonale der Zentralachse (103) zwischen -20° und 20° angeordnet ist, und dieses Flächenelement (24, 34) planar verläuft, konkav oder konvex gekrümmt ist.

12. Knochenanker (10) nach einem der vorhergehenden Ansprüche, **dadurch gekennzeichnet, dass** die Transitionszone (20, 30) wenigstens aus einem Segment (29) besteht, welches wenigstens ein Flächenelement (26, 36) besitzt, welches einen Stauraum definiert, und in diesem Stauraum ein Ring (27, 37) angeordnet ist.

13. Knochenanker (10) nach Anspruch 11, **dadurch gekennzeichnet, dass** der Ring (27, 37) mit der Innenseite (272, 273) den Öffnungsdurchmesser der Transitionszone (d20, d30) definiert.

14. Knochenanker (10) nach einem der Ansprüche 12 oder 13, **dadurch gekennzeichnet, dass** der Ring (27, 37) an seiner radialen Außenseite (271, 371) konvex gekrümmt ist.

15. Knochenanker (10) nach einem der vorhergehenden Ansprüche, **dadurch gekennzeichnet, dass** die Transitionszone (20 und/oder 30) mindestens einen Unterschied, im Strömungswiderstand, oder Druckunterschied, oder inneren Reibung der Flüssigkeit (17), oder Volumenstrom, hervorruft, und dieser Unterschied abhängig von der Fließrichtung der Flüssigkeit (17) ist.

16. Knochenanker (10) nach einem der vorhergehenden Ansprüche, **dadurch gekennzeichnet, dass** die Transitionszone (20 und/oder 30) eine Sperr-Richtung (22) und eine Durchlass-Richtung (21) für Flüssigkeiten besitzt.

17. Knochenanker (10) nach einem der vorhergehenden Ansprüche, **dadurch gekennzeichnet, dass** die Hohlkammer (40) von einer Mittenebene (106) aus betrachtet nach distal an eine Transitionszone (20) und proximal an eine Transitionszone (30) angrenzt und die distale Transitionszone (20) eine Durchlass-Richtung (21) in proximaler Richtung (101) und die proximale Transitionszone (30) eine Durchlass-Richtung (21) in distaler Richtung (102) besitzt.

18. Knochenanker (10) nach einem der vorhergehenden Ansprüche, **dadurch gekennzeichnet, dass** die Hohlkammer (40) von einer Mittenebene (106) aus betrachtet nach distal an eine Transitionszone (20) und proximal an eine Transitionszone (30) angrenzt und die distale Transitionszone (20) eine Sperr-Richtung (22) in distaler Richtung (101) und die proximale Transitionszone (30) eine Sperr-Richtung (22) in proximaler Richtung (102) besitzt.

19. Knochenanker (10) nach einem der vorhergehenden Ansprüche, **dadurch gekennzeichnet, dass** die Hohlkammer (40) mindestens eine seitlich abgehende und mit der Hohlkammer (40) kommunizierende Öffnung (411, 412, 421 oder 422) besitzt, und bei mehr als einer Öffnung die Öffnungen in Umfangsrichtung in ring-artiger Formation (41 und/oder 42) angeordnet sind, und bei mehr als einer in Umfangsrichtung ring-artiger Formation (41 und 42), haben die Öffnungen (411, 412) und (421, 422) je Formation unterschiedliche Öffnungsdurchmesser.

20. Knochenanker (10) nach einem der vorhergehenden Ansprüche, **dadurch gekennzeichnet, dass** der Knochenanker (10) einstückig aufgebaut ist.

21. Knochenanker (10) nach einem der vorhergehenden Ansprüche, **dadurch gekennzeichnet, dass** der Knochenanker (10) durchgängig kanüliert ist.

## Claims

1. Bone anchor (10) for the fixation of bone components and bone fragments, comprising a shaft (13), a neck area (12) and a head (11) located in proximal direction (101) and a tip (14) located in distal direction (102), wherein the bone anchor (10) has a mainly cylindrically shaped hollow chamber (40) with an opening (411, 412, 421, 422) extending laterally and communicating with the hollow chamber, wherein the hollow chamber (40) extending along the central axis (103), wherein the hollow chamber (40) is adjacent to a transition zone (30) located proximally (101) from a central plane (106), **characterized in that** the hollow chamber (40) is mainly provided centrally inside the bone anchor (10) in distal-proximal alignment and the transition zone (30)has at least partially an inner diameter (d30) and the hollow chamber (40) has at least partially an opening diameter (d40), and the opening diameter of the hollow chamber (d40) is greater than the inner diameter (d30) of the proximal transition zone (30).

2. Bone anchor according to claim 1, **characterized in that** in the further proximal course (101) along the central axis (103) an opening (16) adjoins the proximal transition zone (30), which is suitable for receiving a cannula at least in sections.

3. A bone anchor (10) according to claim 2, **characterized in that** the opening diameter (d16) of the opening (16) adjacent to the proximal transition zone (30) is greater than at least one portion of the inner diameter (d30) of the proximal transition zone (30).

4. Bone anchor (10) according to one of the preceding claims, **characterized in that** the opening (16) opens in proximal direction (101) into a tool attachment point (19).

5. Bone anchor (10) according to one of the preceding claims, **characterized in that** the hollow chamber (40) adjoins a transition zone (20) located distally from a central axis (106), and the distal transition zone (20) has at least in sections an internal diameter (d20), and the hollow chamber (40) has at least in sections an opening diameter (d40), and the opening diameter of the hollow chamber (d40) is greater than the internal diameter (d20) of the distal transition zone (20).

6. Bone anchor (10) according to one of the preceding claims, **characterized in that** the transition zone (20, 30) defines a section which causes a higher flow resistance compared to a section of the same length of the hollow chamber (40).

7. Bone anchor (10) according to one of the preceding claims, **characterized in that** the transition zone (20, 30) defines a section that causes a higher pressure difference in the liquid (17) compared to a section of the same length in the hollow chamber (40).

8. Bone anchor (10) according to one of the preceding claims, **characterized in that** the transition zone (20, 30) defines a section which leads to a higher internal friction in the liquid (17) compared to a section of the same length of the hollow chamber (40).

9. Bone anchor (10) according to any of the preceding claims, **characterized in that** the transition zone (20, 30) has a higher surface friction coefficient or a higher surface roughness than the hollow chamber (40).

10. Bone anchor (10) according to one of the preceding claims, **characterized in that** the transition zone (20, 30) allows a smaller volume flow of liquid (17) than the hollow chamber (40).

11. Bone anchor (10) according to one of the preceding claims, **characterized in that** the transition zone (20, 30) consists of at least one segment (29) having at least one surface element (25, 35, 272, 273) defining the inner opening diameter (d20, d30) and at least one surface element (26, 36) defining a stagnation space, and at least one surface element (24, 34) which generates a dynamic pressure in the blocking direction (22), in which the surface element (24, 34) is arranged at an angle to an orthogonal of the central axis (103) between -20° and 20°, and this surface element (24, 34) is planar, concave or convexly curved.

12. Bone anchor (10) according to one of the preceding claims, **characterized in that** the transition zone (20, 30) consists of at least one segment (29) having at least one surface element (26, 36) defining a stagnation space, and a ring (27, 37) is arranged in this stagnation space.

13. Bone anchor (10) according to claim 11, **characterized in that** the ring (27, 37) with the inner side (272, 273) defines the opening diameter of the transition zone (d20, d30).

14. Bone anchor (10) according to claims 12 or 13, **characterized in that** the ring (27, 37) is convexly curved at its radial outside (271, 371).

15. Bone anchor (10) according to one of the preceding claims, **characterized in that** the transition zone (20 and/or 30) causes at least one difference in the flow resistance, or pressure difference, or internal friction of the liquid (17), or volume flow, and this difference is dependent on the flow direction of the liquid (17).

16. Bone anchor (10) according to one of the preceding claims, **characterized in that** the transition zone (20 and/or 30) has a blocking direction (22) and a forward direction (21) for liquids.

17. Bone anchor (10) according to one of the preceding claims, **characterized in that** the hollow chamber (40), as viewed from a central plane (106), is distally adjacent to a transition zone (20) and proximally adjacent to a transition zone (30), and the distal transition zone (20) has a forward direction (21) in proximal direction (101) and the proximal transition zone (30) has a forward direction (21) in distal direction (102).

18. A bone anchor (10) according to any one of the preceding claims, **characterized in that** the hollow chamber (40), as viewed from a central plane (106), is distally adjacent to a transition zone (20) and proximally adjacent to a transition zone (30), and the distal transition zone (20) has a blocking direction (22) in distal direction (101) and the proximal transition zone (30) has a blocking direction (22) in proximal direction (102).

19. Bone anchor (10) according to any of the preceding claims, **characterized in that** the hollow chamber (40) has at least one laterally extending opening (411, 412, 421 or 422) communicating with the hollow chamber (40), and in the case of more than one opening, the openings are arranged circumferentially in ring-like formation (41 and/or 42), and in the case of more than one circumferentially ring-like formation (41 and 42), the openings (411, 412) and (421, 422) have different opening diameters per formation.

20. Bone anchor (10) according to one of the preceding claims, **characterized in that** the bone anchor (10) is formed in one piece.

21. Bone anchor (10) according to one of the preceding claims, **characterized in that** the bone anchor (10) is grooved throughout.

## Revendications

1. Ancrage osseux (10) pour la fixation de composants osseux et de fragments osseux, composé d'une tige (13), d'une zone de cou (12) et d'une tête (11) située dans la direction proximale (101), ainsi que d'une pointe (14) située dans la direction distale (102), l'ancrage osseux (10) présentant une chambre creuse (40) de forme principalement cylindrique avec une ouverture (411, 412, 421, 422) se développant latéralement et communiquant avec la chambre creuse, la chambre creuse (40) s'étendant le long de l'axe central (103), la chambre creuse (40) étant adjacente à une zone de transition (30) proximale (101) située à partir d'un plan médian (106), **caractérisé en ce que** la chambre creuse (40) est prévue dans une orientation distale-proximale principalement centrale à l'intérieur de l'ancrage osseux (10) et la zone de transition (30) présente au moins partiellement un diamètre intérieur (d30) et la chambre creuse (40) présente au moins partiellement un diamètre d'ouverture (d40) et le diamètre d'ouverture de la chambre creuse (d40) est supérieur au diamètre intérieur (d30) de la zone de transition proximale (30).

2. Ancrage osseux selon la revendication 1, **caractérisé en ce que** dans le tracé proximal supplémentaire (101) le long de l'axe central (103), une ouverture (16) est adjacente à la zone de transition proximale (30), qui est adaptée pour recevoir une canule au moins partiellement.

3. Ancrage osseux (10) selon la revendication 2, **caractérisé en ce que** le diamètre d'ouverture (d16) de l'ouverture (16) adjacente à la zone de transition proximale (30) est supérieur à au moins une partie du diamètre intérieur (d30) de la zone de transition proximale (30).

4. Ancrage osseux (10) selon l'une des revendications précédentes, **caractérisé en ce que** l'ouverture (16) débouche en direction proximale (101) dans un point d'attache d'outil (19).

5. Ancrage osseux (10) selon l'une des revendications précédentes, **caractérisé en ce que** la chambre creuse (40) est adjacente à une zone de transition (20) située de manière distale à partir d'un plan médian (106), et la zone de transition distale (20) présente au moins partiellement un diamètre intérieur (d20) et la chambre creuse (40) présente au moins partiellement un diamètre d'ouverture (d40), et le diamètre d'ouverture de la chambre creuse (d40) est supérieur au diamètre intérieur (d20) de la zone de transition distale (20).

6. Ancrage osseux (10) selon l'une des revendications précédentes, **caractérisé en ce que** la zone de transition (20, 30) définit un tronçon qui, par rapport à un tronçon de même longueur de la chambre creuse (40), provoque une résistance plus élevée à l'écoulement.

7. Ancrage osseux (10) selon l'une des revendications précédentes, **caractérisé en ce que** la zone de transition (20, 30) définit un tronçon qui, par rapport à un tronçon de même longueur de la chambre creuse (40), provoque une différence de pression plus élevée dans le fluide (17).

8. Ancrage osseux (10) selon l'une des revendications précédentes, **caractérisé en ce que** la zone de transition (20, 30) définit un tronçon qui, par rapport à un tronçon de même longueur de la chambre creuse (40), conduit à un frottement interne plus élevé dans le fluide (17).

9. Ancrage osseux (10) selon l'une des revendications précédentes, **caractérisé en ce que** la zone de transition (20, 30) a un coefficient de frottement de surface plus élevé ou une rugosité de surface plus élevée que la chambre creuse (40).

10. Ancrage osseux (10) selon l'une des revendications précédentes, **caractérisé en ce que** la zone de transition (20, 30) permet un débit volumique de fluide (17) inférieur à celui de la chambre creuse (40).

11. Ancrage osseux (10) selon l'une des revendications précédentes, **caractérisé en ce que** la zone de transition (20, 30) se compose d'au moins un segment (29) qui possède au moins un élément de surface (25, 35, 272, 273) qui définit le diamètre intérieur de l'ouverture (d20, d30), et au moins un élément de surface (26, 36) qui définit un espace de retenue, et au moins un élément de surface (24, 34) qui génère une pression dynamique dans la direction de blocage (22), dans lequel l'élément de surface (24, 34) est disposé à un angle par rapport à une orthogonale de l'axe central (103) entre -20 ° et 20 °, et cet élément de surface (24, 34) est plan, concave ou courbée de façon convexe.

12. Ancrage osseux (10) selon l'une des revendications précédentes, **caractérisé en ce que** la zone de transition (20, 30) se compose d'au moins un segment (29) qui possède au moins un élément de surface (26, 36) qui définit un espace de retenue, et une bague (27, 37) est disposée dans cet espace de retenue.

13. Ancrage osseux (10) selon la revendication 11, **caractérisé en ce que** la bague (27, 37) définit avec la face intérieure (272, 273) le diamètre d'ouverture de la zone de transition (d20, d30).

14. Ancrage osseux (10) selon l'une des revendications 12 ou 13, **caractérisé en ce que** la bague (27, 37) est courbée de manière convexe sur sa face externe radiale (271, 371).

15. Ancrage osseux (10) selon l'une des revendications précédentes, **caractérisé en ce que** la zone de transition (20 et/ou 30) provoque au moins une différence de résistance à l'écoulement, ou de différence de pression, ou de frottement interne du fluide (17), ou de débit volumique, et cette différence est fonction du sens d'écoulement du fluide (17).

16. Ancrage osseux (10) selon l'une des revendications précédentes, **caractérisé en ce que** la zone de transition (20 et/ou 30) possède une direction de blocage (22) et une direction de passage (21) pour les fluides.

17. Ancrage osseux (10) selon l'une des revendications précédentes, **caractérisé en ce que** la chambre creuse (40), vue depuis un plan médian (106), est adjacente distalement à une zone de transition (20) et proximalement à une zone de transition (30), et la zone de transition distale (20) a une direction de passage (21) dans la direction proximale (101) et la zone de transition proximale (30) a une direction de passage (21) dans la direction distale (102).

18. Ancrage osseux (10) selon l'une des revendications précédentes, **caractérisé en ce que** la chambre creuse (40), vue depuis un plan médian (106), est adjacente distalement à une zone de transition (20) et proximalement à une zone de transition (30), et la zone de transition distale (20) a une direction de blocage (22) dans la direction distale (101) et la zone de transition proximale (30) a une direction de blocage (22) dans la direction proximale (102).

19. Ancrage osseux (10) selon l'une des revendications précédentes, **caractérisé en ce que** la chambre creuse (40) possède au moins une ouverture (411, 412, 421 ou 422) se développant latéralement et communiquant avec la chambre creuse (40), et **en ce que** dans le cas de plus d'une ouverture, les ouvertures sont disposées dans la direction circonférentielle dans une formation de type annulaire (41 et/ou 42), et dans le cas de plus d'une formation de type annulaire (41 et 42) dans la direction circonférentielle, les ouvertures (411, 412) et (421, 422) ont des diamètres d'ouverture différents par formation.

20. Ancrage osseux (10) selon l'une des revendications précédentes, **caractérisé en ce que** l'ancrage osseux (10) est construit en un seul tenant.

21. Ancrage osseux (10) selon l'une des revendications précédentes, **caractérisé en ce que** l'ancrage osseux (10) est canulé de part en part.
